(19) Europäisches Patentamt
European Patent Office
Office européen des brevets

(11) Veröffentlichungsnummer: **0 227 982**
**A1**

(12) **EUROPÄISCHE PATENTANMELDUNG**

(21) Anmeldenummer: **86117011.6**

(22) Anmeldetag: **08.12.86**

(51) Int. Cl.⁴: **C 07 D 513/04**, A 61 K 31/54
// (C07D513/04, 279:00, 221:00)

(30) Priorität: **19.12.85 DE 3545097**

(43) Veröffentlichungstag der Anmeldung: **08.07.87**
**Patentblatt 87/28**

(84) Benannte Vertragsstaaten: **AT BE CH DE ES FR GB IT LI NL SE**

(71) Anmelder: **BAYER AG, Konzernverwaltung RP Patentabteilung, D-5090 Leverkusen 1 Bayerwerk (DE)**

(72) Erfinder: **Klausener, Alexander, Dr., Dahlerdyk 173, D-4150 Krefeld (DE)**
Erfinder: **Fengler, Gerd, Dr., Bethelstrasse 16, D-4150 Krefeld (DE)**
Erfinder: **Buysch, Hans-Josef, Dr., Brandenburger Strasse 28, D-4150 Krefeld (DE)**
Erfinder: **Pelster, Bernhard, Dr., Pleisufer 6a, D-5205 St. Augustin 1 (DE)**

(54) **1-H-Pyrido-[3,2-b][1,4]-thiazine.**

(57) 1-H-Pyrido-[3,2-b][1,4]-thiazine der Formel

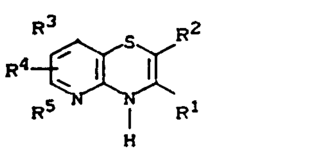

I

in der
$R^1$ Wasserstoff, gegebenenfalls substituiertes Alkyl oder Alkenyl oder die Gruppe

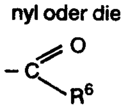

in der
$R^6$ für gegebenenfalls substituiertes Alkyl, Aryl oder Alkoxy steht,
bedeutet,
$R^2$ Wasserstoff, Nitril, die Gruppe

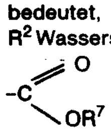

in der $R^7$ für gegebenenfalls substituiertes Alkyl oder Aryl steht,
oder die Gruppe

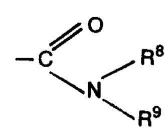

in der
$R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6gliedrigen, gegebenenfalls substituierten Heterocyclus verbunden sein können,
oder die Gruppe

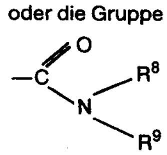

in der $R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6gliedrigen, gegebenenfalls substituierten Heterocyclus verbunden sein können,
oder die Gruppe

EP 0 227 982 A1

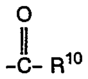

in der
$R^{10}$ für gegebenenfalls substituiertes Alkyl steht, wobei $R^1$ und $R^{10}$ einen gegebenenfalls substituierten 5- bis 8gliedrigen Carbocyclus bilden können,
oder gegebenenfalls substituiertes Aryl bedeutet, und $R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

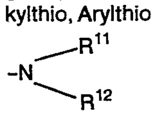

in der
$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6gliedrigen gegebenenfalls substituierten Heterocyclus verbunden sein können, bedeuten, können durch Umsetzung von 2-Amino-3-pyridinthiolen mit α-Halogencarbonylverbindungen hergestellt werden. Die neuen Verbindungen können als Wirkstoffe in Arzneimitteln verwendet werden.

 0227982

BAYER AKTIENGESELLSCHAFT 5090 Leverkusen, Bayerwerk
Konzernverwaltung RP
Patentabteilung Mn/em-c

1-H-Pyrido-[3,2-b][1,4]-thiazine

Die vorliegende Erfindung betrifft neue 1-H-Pyrido-[3,2-b][1,4]-thiazine und deren Salze, Verfahren zu ihrer Herstellung und ihre Verwendung in Arzneimitteln.

Es ist bekannt, daß die durch das Enzym Lipoxygenase gebildeten Metaboliten der Arachidonsäure an der Entstehung von entzündlichen und allergischen Prozessen beteiligt sind [Goetzl, Immunology 40 709 (1980); Ford-Hutchinson, J. Pharm. Pharmacol. 32 517 (1980) und Nature 286 264 (1980; Samuelsson, Trends in Pharmacol. Sci. May 1980 227; Borgeat, J. Med. Chem. 24 121 (1981)].

Es wurden neue 1-H-Pyrido-[3,2-b][1,4]-thiazine der Formel

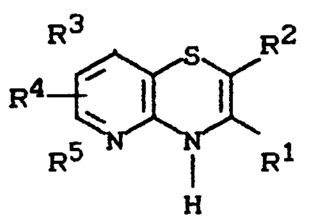

(I),

gefunden, in der

$R^1$ Wasserstoff, gegebenenfalls substituiertes Alkyl oder Alkenyl oder die Gruppe

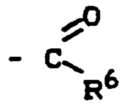

in der

$R^6$ für gegebenenfalls substituiertes Alkyl, Aryl oder Alkoxy steht,

bedeutet,

$R^2$ Wasserstoff, Nitril, die Gruppe

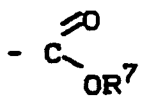

in der $R^7$ für gegebenenfalls substituiertes Alkyl oder Aryl steht,

oder die Gruppe

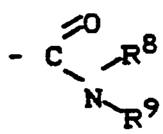

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Heterocyclus verbunden sein können,

oder die Gruppe

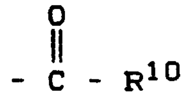

in der

$R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

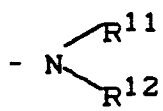

in der

$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen

gegebenenfalls substituierten Heterocyclus verbunden sein können,

bedeuten,

und deren Salze.

Überraschenderweise sind die neuen 1-H-Pyrido-[3,2-b]-[1,4]-thiazine starke Lipoxygenasehemmer. Überraschenderweise hemmen sie die Lipoxygenase sehr spezifisch bereits in solchen Konzentrationen, bei denen die Cyclooxygenase nicht beeinflußt wird. Diese sehr starke und spezifische Wirkung der 1-H-Pyrido-[3,2-b][1,4]-thiazine konnte nicht erwartet werden.

Alkyl steht im allgemeinen für geradkettige oder verzweigte Kohlenwasserstoffreste mit 1 bis 8 Kohlenstoffatomen. Bevorzugt wird Niederalkyl mit 1 bis 6 Kohlenstoffatomen. Beispielsweise seien Methyl, Ethyl, Propyl, Isopropyl, Butyl, Isobutyl, Pentyl, Isopentyl, Hexyl und Isohexyl genannt.

Alkenyl steht im allgemeinen für einen ungesättigten, geradkettigen oder verzweigten Kohlenwasserstoffrest mit 1 bis 8 Kohlenstoffatomen und einer oder zwei, bevorzugt einer, Doppelbindung. Bevorzugt wird Niederalkenyl mit 2 bis etwa 6 Kohlenstoffatomen. Beispielsweise sei Vinyl genannt.

In den Alkoxycarbonyl-, Säureamid- und Alkylcarbonylresten hat Alkyl im allgemeinen den oben genannten Bedeutungsumfang.

Als Alkoxycarbonyl seien beispielsweise genannt: Methoxycarbonyl, Ethoxycarbonyl, Isopropoxycarbonyl, Isopentoxycarbonyl.

Als Säureamidreste seien beispielsweise genannt: Methylamid, t- Butylamid, Morpholinid, p-Methoxyanilid.

Als Alkylcarbonylreste seien beispielsweise genannt: Methylcarbonyl, Butylcarbonyl, Isopentylcarbonyl, Isohexylcarbonyl.

Für den Fall, daß $R^1$ und $R^{10}$ zu einem 5- bis 8-gliedrigen gegebenenfalls substituierten Carbocyclus verbunden sind, sei als Verbindungsglied eine gegebenenfalls durch Niederalkyl substituierte 2 bis 5 Kohlenstoffatome enthaltende Kohlenwasserstoffkette genannt. Als auf diese Weise gebildete Carbocyclen seien beispielsweise genannt: Cyclo-pent-2-en-1-on, Cyclo-hex-2-en-1-on und 5,5-Dimethyl-cyclo-hex-2-en-1-on.

Aryl steht im allgemeinen für einen aromatischen Rest mit 6 bis etwa 12 Kohlenstoffatomen. Bevorzugte Arylreste sind Phenyl, Naphthyl, Diphenyl, bevorzugt Phenyl.

Alkoxy steht im allgemeinen für einen über ein Sauerstoffatom gebundenen geradkettigen oder verzweigten Kohlenwasserstoffrest mit 1 bis 8 Kohlenstoffatomen. Bevorzugt ist Niederalkoxy mit 1 bis etwa 6 Kohlenstoffatomen. Beispielsweise seien Methoxy, Ethoxy, Propoxy, Isopropoxy, Butoxy, Isobutoxy, Pentoxy, Isopentoxy, Hexoxy und Isohexoxy genannt.

Aryloxy steht im allgemeinen für einen über Sauerstoff gebundenen Arylrest mit 6 bis 12 Kohlenstoffatomen. Bevorzugt ist Phenyloxy.

Alkylthio steht im allgemeinen für einen über ein Schwefelatom gebundenden, geradkettigen oder verzweigten Kohlenwasserstoffrest mit 1 bis 8 Kohlenstoffatomen. Bevorzugt ist Niederalkylthio mit 1 bis etwa 6 Kohlenstoffatomen. Beispielsweise seien Ethylthio, Butylthio, tert.-Butylthio und Hexylthio genannt.

Arylthio steht im allgemeinen für einen über ein Schwefelatom gebundenen Arylrest mit 6 bis 12 Kohlenstoffatomen. Bevorzugt seien [m-(Trifluormethyl)phenyl]thio, [p-(Fluor)phenyl]thio, [p-(t-Butyl)phenyl]thio genannt.

Die Reste $R^8$ und $R^9$ und $R^{11}$ und $R^{12}$ können durch eine Kohlenwasserstoffbrücke, die gegebenenfalls noch durch weitere Heteroatome unterbrochen sein kann, zu einem 5- oder 6-gliedrigen gegebenenfalls substituierten Heterocyclus verbunden sein. Als Heteroatome seien eine oder zwei Sauerstoff- und/oder Stickstoff- und/oder Schwefelatome genannt. Bevorzugt seien hier Pyrrolyl-, Pyrimidyl, Imidazolyl, Morpholinyl und N-Methyl-piperazinyl genannt.

Halogen bedeutet im allgemeinen Fluor, Chlor, Brom und Iod, bevorzugt Fluor, Chlor und Brom.

Die genannten Reste können gegebenenfalls substituiert sein. Als Substituenten seien beispielsweise genannt Halogen, bevorzugt Fluor, Chlor und Brom, Nitro, Cyano, Carboxymethyl, Carboxyethyl und Trifluormethyl.

Die erfindungsgemäßen 1-H-Pyrido-[3,2-b][1,4]-thiazine können auch in Form ihrer Salze vorliegen. Im allgemeinen seien hier Salze mit organischen oder anorganischen Säuren genannt.

Im Rahmen der vorliegenden Erfindung werden physiologisch unbedenkliche Salze bevorzugt. Physiologisch unbedenkliche Salze der 1-H-Pyrido-[3,2-b][1,4]-thiazine sind bevorzugt Salze mit anorganischen Säuren wie beispielsweise Hydrochloride, Hydrobromide, Sulfate, Hydrogensulfate, Phosphate oder Hydrogenphosphate oder mit organischen Säuren wie beispielsweise Formiate, Acetate, Benzoate, Maleate, Fumarate, Tartrate, Nitrate oder Lactate.

Es werden 1-H-Pyrido-[3,2-b][1,4]-thiazine der Formel (I) bevorzugt, bei denen

$R^1$ Wasserstoff, gegebenenfalls substituiertes Alkyl ($C_1$ bis $C_8$) oder Alkenyl ($C_2$ bis $C_8$) oder die Gruppe

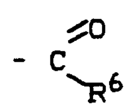

in der $R^6$ für gegebenenfalls substituiertes Alkyl ($C_1$ bis $C_8$), Alkoxy ($C_1$ bis $C_8$) oder Aryl ($C_6$ bis $C_{12}$) steht,

bedeutet,

$R^2$ Wasserstoff, Nitril, die Gruppe

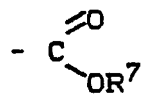

in der $R^7$ für gegebenenfalls substituiertes Alkyl ($C_1$ bis $C_8$) steht,

oder die Gruppe

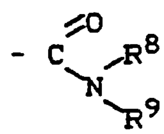

in der

$R^8$ und $R^9$ gleich oder verschieden sind und für Wasserstoff, gegebenenfalls substituiertes Alkyl ($C_1$ bis $C_8$) oder Aryl ($C_6$ bis $C_{12}$) stehen und wobei die Reste durch eine Kohlenwasserstoffbrücke, welche gegebenenfalls durch ein oder zwei Sauerstoff- und/oder Stickstoff- und/oder Schwefelatome unterbrochen sein kann, zu einem 5-oder 6-gliedrigen, gegebenenfalls substituierten Heterocyclus verbunden sein können,

oder die Gruppe

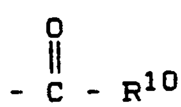

in der $R^{10}$ für gegebenenfalls substituiertes Alkyl ($C_1$ bis $C_8$) steht,

wobei $R^1$ und $R^{10}$ einen gegebenenfalls substituierten 5- bis 8-gliedrigen Carbocyclus bilden können,

oder gegebenenfalls substituiertes Aryl ($C_6$ bis $C_{12}$) bedeutet,

$R^3$ Wasserstoff bedeutet,

und

$R^4$ und $R^5$ gleich oder verschieden sind und Wasserstoff, Halogen, gegebenenfalls substituiertes Alkyl ($C_1$ bis $C_8$), Alkoxy ($C_1$ bis $C_8$), Aryloxy ($C_6$ bis $C_{12}$), Alkylthio ($C_1$ bis $C_8$), Arylthio ($C_6$ bis $C_{12}$) oder die Gruppe

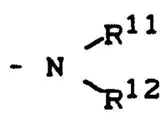

in der

$R^{11}$ und $R^{12}$ gleich oder verschieden sind und für Wasserstoff, gegebenenfalls substituiertes Alkyl ($C_1$ bis $C_8$) oder Aryl ($C_6$ bis $C_{12}$) stehen und wobei die Reste durch eine Kohlenwasserstoffbrücke, welche gegebenenfalls durch ein oder zwei Sauerstoff- und/oder Stickstoff- und/oder Schwefelatome unterbrochen sein kann, zu einem 5-oder 6-gliedrigen gegebenenfalls substituierten Heterocyclus verbunden sein können,

bedeuten

und deren Salze.

Besonders bevorzugt werden 1-H-Pyrido-[3,2-b][1,4]-thiazine der Formel (I), bei denen

$R^1$ Wasserstoff, gegebenenfalls durch Carboxymethyl oder Carboxyethyl substituiertes Niederalkyl, Vinyl oder Carboxymethyl oder Carboxyethyl bedeutet,

$R^2$ Wasserstoff, Carboxymethyl, Carboxyethyl oder die Gruppe

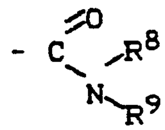

in der

$R^8$ und $R^9$ gleich oder verschieden sind und für Wasserstoff, Niederalkyl, Phenyl stehen und wobei die Reste zu einem Piperidyl-, Morpholinyl-, Piperazinyl- oder N-Methylpiperazinylrest verbunden sein können,

oder die Gruppe

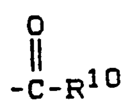

in der

$R^{10}$ Methyl oder Ethyl bedeutet und

wobei $R^1$ und $R^{10}$ einen gegebenenfalls durch eine oder zwei Methylgruppen substituierten 5- bis 8-gliedrigen Carbocyclus bilden können,

bedeutet,

$R^3$ und $R^4$ Wasserstoff, Niederalkyl oder Halogen bedeuten,

und

$R^5$ Wasserstoff, Fluor, Chlor, Brom, Niederalkyl, Niederalkoxy, Piperidyl, Morpholinyl oder N-Methylpiperazinyl bedeutet,

und deren Salze.

Beispielsweise seien die folgenden 1-H-Pyrido-[3,2-b]-[1,4]-thiazine genannt:

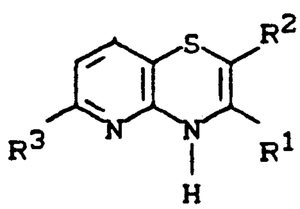

| $R^1$ | $R^2$ | $R^3$ |
|---|---|---|
| H | COOMe | Et |
| H | COOBu | Me |
| H | $COOBu^t$ | H |
| Me | COOMe | Et |
| Me | COOBu | Me |
| Me | $COOBu^t$ | H |
| $Bu^t$ | COOMe | Et |
| $Bu^t$ | COOBu | Me |
| $Bu^t$ | $COOBu^t$ | H |
| Me | COOEt | F |
| Me | $COOPr^i$ | Cl |
| Me | $COOBu^t$ | Br |

0227982

| $R^1$ | $R^2$ | $R^3$ |
|---|---|---|
| H | $CONH_2$ | OEt |
| Me | CONHMe | O(p-Cl-Ph) |
| Et | CONHBu | O(o-CN-Ph) |
| Pr | CONPh | $O(m-NO_2-Ph)$ |
| $Pr^i$ | $CONHCH_2CH_2OH$ | SBu |
| Bu | $CONHCH_2CH_2OMe$ | S(p-Cl-Ph) |
| $Bu^i$ | $CONCH_2CH_2NH_2$ | S(o-OMe-Ph) |
| $Bu^t$ | $CONCH_2CH_2NHEt$ | $S(m-NO_2-Ph)$ |
| H | $CONHCH_2CH_2NMe_2$ | $NMe_2$ |
| Me | $CONMe_2$ | $NEt_2$ |
| Et | CONMeEt | NMePh |
| Me | CN | Me |
| COOEt | COOEt | Me |
| COOBu | COOBu | Me |
| COOEt | H | H |
| $CH_2COOEt$ | H | H |
| $CH_2COOBu^t$ | H | H |
| Me | COOEt | $N(CH_2CH_2OMe)_2$ |
| Me | COOEt | $N(CH_2CH_2NMe_2)_2$ |
| Et | $COOCH_2CHF_2$ | NEt(p-F-Ph) |
| Me | $COOCH_2CH_2Cl$ | $NMe(m-NO_2-Ph)$ |
| H | $COOCH_2CN(Me)OMe$ | NMe(p-Me-Ph) |
| $Bu^t$ | $COOCH_2CH_2CH_2OMe$ | NMe(p-Cl-Ph) |
| $CH=CH_2$ | H | H |
| H | Ph | H |
| H | p-Cl-Ph | H |
| H | p-OMe-Ph | H |
| H | m-CN-Ph | Me |
| H | $m-NO_2-Ph$ | Cl |
| H | o-Br-Ph | Br |

| $R^1$ | $R^2$ | $R^3$ |
|---|---|---|
| H | o-F-Ph | F |
| H | CO-Me | H |
| H | CO-Et | OEt |
| Me | CON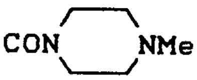 | Cl |
| Me | CON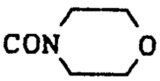 | F |
| Me | CON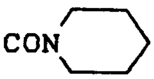 | OEt |
| Me | CON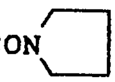 | SPh |
| H | COOBu | 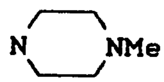 |
| H | COOBu | 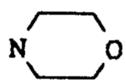 |
| H | $CONEt_2$ | 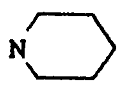 |
| H | $CONEt_2$ | 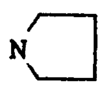 |

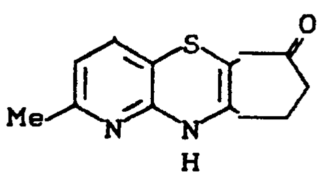

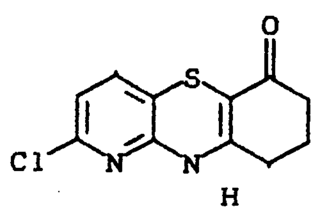

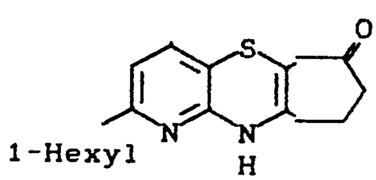

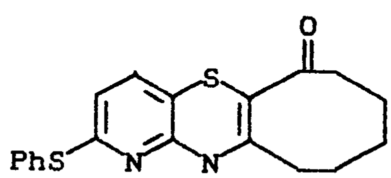

Es wurde auch ein Verfahren zur Herstellung von 1-H-Pyrido-[3,2-b][1,4]-thiazinen gefunden, das dadurch gekennzeichnet ist, daß man 2-Amino-3-pyridinthiole der Formel

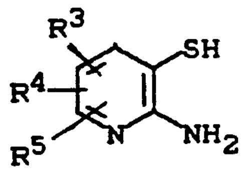

(II),

in der

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

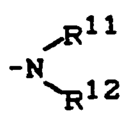

in der

$R^{11}$ und $R^{12}$ gleich oder verschieden sind und Wasserstoff, gegebenenfalls substituiertes Alkyl oder Aryl stehen, und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Heterocyclus verbunden sein können,

bedeuten,

mit einer α-Halogencarbonylverbindung der Formel

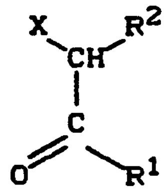

(III),

in der

X Halogen bedeutet,

$R^1$ Wasserstoff, gegebenenfalls substituiertes Alkyl oder Alkenyl oder die Gruppe

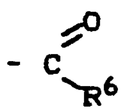

in der $R^6$ für gegebenenfalls substituiertes Alkyl, Alkoxy oder Aryl steht,

bedeutet und

$R^2$ Wasserstoff, Nitril, die Gruppe

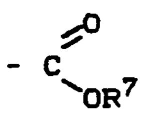

in der $R^7$ für gegebenenfalls substituiertes Alkyl oder Aryl steht,

oder die Gruppe

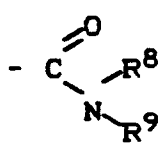

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke die gegebenenfalls noch durch weitere Heteroatome unterbrochen sein kann, zu einem 5- oder 6-gliedrigen, gegebenenfalls substituierten Heterocyclus verbunden sein können,

oder die Gruppe

$$-\overset{\overset{\displaystyle O}{\|}}{C}-R^{10}$$

in der $R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

in Gegenwart einer Base umsetzt.

Das erfindungsgemäße Verfahren kann durch die folgende Reaktionsgleichung erläutert werden

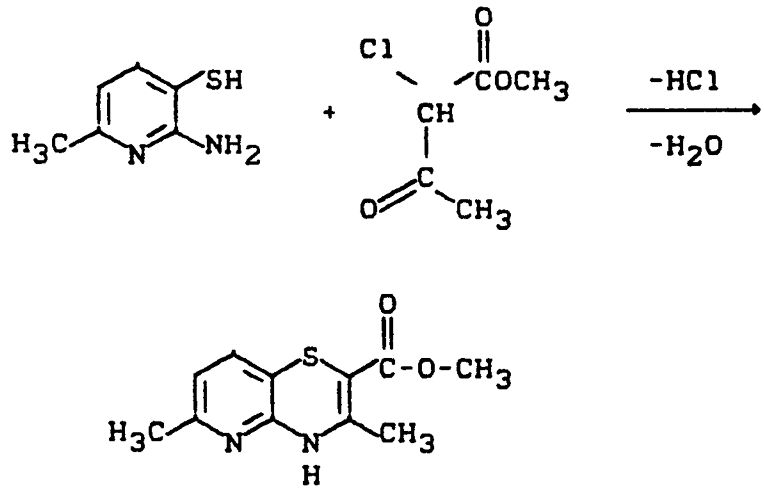


2-Amino-3-pyridinthiole für das erfindungsgemäße Verfahren können aus acylierten 2-Pyridinaminen und beispielsweise Dibenzyldisulfid hergestellt werden. Für das erfindungsgemäße Verfahren ist es nicht erforderlich, die 2-Amino-3-pyridinthiole in gereinigter Form einzusetzen. Sie können auch in Form ihrer Salze, insbesondere ihrer Alkali-, Erdalkali- oder Ammoniumsalze verwendet werden. Besonders vorteilhaft ist die Verwendung dieser Thiolate in der bei ihrer Herstellung anfallenden Rohform.

α-Halogencarbonylverbindungen für das erfindungsgemäße Verfahren sind an sich bekannt (J. Heterocycl. Chem. 10, 938 (1973); Org. Syntheses 21, 4 (1941); Ann. Chem. 439, 211 (1924)) und können beispielsweise durch Umsetzung von Carbonylverbindungen mit Sulfurylchlorid hergestellt

werden. Als α-Halogencarbonylverbindungen seien die Fluor-, Chlor-, Brom- oder Jodverbindungen, bevorzugt die Chlor-Verbindungen, genannt.

Das erfindungsgemäße Verfahren wird in Gegenwart einer Base durchgeführt. Dafür kommen anorganische Basen wie Alkali- oder Erdalkalimetallhydroxide sowie -Carbonate bzw. entsprechende Ammoniumverbindungen, aber auch organische Basen wie tertiäre Amine (Trialkyl($C_1$-$C_6$)-Amine) oder Heterocyclen (beispielsweise Pyridin, Morpholin und Chinolin) in Frage.

Das erfindungsgemäße Verfahren wird vorzugsweise in Gegenwart eines Verdünnungsmittels durchgeführt. Als Verdünnungsmittel können alle inerten Lösungsmittel eingesetzt werden, die sich unter den Reaktionsverbindungen nicht verändern. Hierzu gehören vorzugsweise Ether, wie Dioxane, Tetrahydrofuran, dipolare aprotische Solventien, wie Dimethylsulfid, Dimethylformamid und N-Methylpyrrolidon, Wasser, und, besonders bevorzugt, niedere Alkohle, wie Ethanol, Methanol, n- und i-Propanol sowie n-, i- und t-Butanol.

Das erfindungsgemäße Verfahren kann durchgeführt werden in Gegenwart von ausschließlich einem oder mehreren organischen Lösungsmitteln, im Gemisch solcher Lösungsmittel mit Wasser oder in einem oder mehreren nicht mit Wasser mischbaren Lösungsmitteln.

Das erfindungsgemäße Verfahren wird im allgemeinen im Temperaturbereich von etwa -10° C bis etwa +100° C, vorzugsweise im Temperaturbereich von 0° C bis 60° C, durchgeführt.

Das erfindungsgemäße Verfahren kann unter Normaldruck, jedoch auch unter erhöhtem oder vermindertem Druck (beispielsweise im Bereich von 0,5 bis 10,0 bar) durchgeführt werden. Bevorzugt arbeitet man unter Normaldruck.

Es kann sich als zweckmäßig erweisen, die gesamte Umsetzung oder einzelne Phasen der Umsetzung unter Ausschluß von Luftsauerstoff durchzuführen. Bevorzugt geschieht dies durch Arbeiten unter Stickstoff oder Argon.

Das erfindungsgemäße Verfahren kann beispielsweise wie folgt durchgeführt werden: Man setzt die 2-Amino-3-pyridinthiole und die α-Halogencarbonylverbindungen in Gegenwart einer Base im Molverhältnis von etwa 1 : 1 um. Es kann sich als zweckmäßig erweisen, in einzelnen Fällen Überschüsse der einen oder anderen Komponenten zu verwenden.

Nach der in Gegenwart der Base durchgeführten Umsetzung der 2-Amino-3-pyridinthiole mit den α-Halogencarbonylverbindungen neutralisiert man das Reaktionsgemisch oder säuert das Reaktionsgemisch an. Bevorzugt werden dazu anorganische Säuren, z.B. Salzsäure, Schwefelsäure, Salpetersäure, aber auch organische Säuren, wie Essigsäure oder auch geeignete saure Ionenaustauscher verwendet.

Die genannten Säuren können allein oder im Gemisch mit anderen Säuren verwendet werden sowie in reiner oder in verdünnter Form. Als Verdünnungsmittel kommen alle inerten, organischen oder auch anorganischen Lösungsmittel in Frage.

Das Reaktionsgemisch wird zur Isolierung der erfindungsgemäßen 1-H-Pyrido-[3,2-b][1,4]-thiazine in an sich bekannter Art aufgearbeitet. Die Reinigung der Reaktionsprodukte erfolgt durch Umkristallisieren oder mit Hilfe chromatographischer Verfahren (Nieder-, Mittel- und Hochdruck-Flüssigchromatographie).

Eine besondere Ausführungsform des erfindungsgemäßen Verfahrens zur Herstellung von 1-H-Pyrido-[3,2-b][1,4]-thiazinen ist dadurch gekennzeichnet, daß man zuerst 2-Pyridin-Amine der Formel

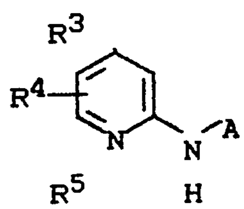

(IV),

in der

A für einen Acylrest der Formel

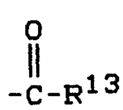 oder 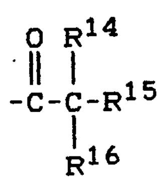 oder 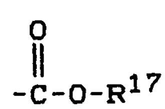

wobei

$R^{13}$ für gegebenenfalls substituiertes Phenyl steht und

$R^{14}$ bis $R^{17}$ gleich oder verschieden sind und für Niederalkyl oder gegebenenfalls substituiertes Phenyl stehen,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

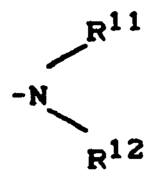

in der

$R^{11}$ und $R^{12}$ gleich oder verschieden sind und Wasserstoff, gegebenenfalls substituiertes Alkyl oder Aryl stehen, und wobei die Reste durch eine Kohlenwasserstoffbrücke, die gegebenenfalls durch weitere Heteroatome unterbrochen sein kann, zu einem 5-oder 6-gliedrigen, gegebenenfalls substituierten Heterocyclus verbunden sein können,

bedeuten,

in Gegenwart von metallorganischen Verbindungen und Disulfiden der Formel

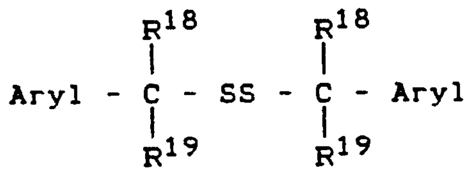 (V),

in der

der Rest 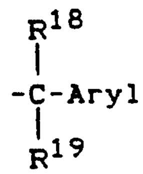 eine Thiol-Schutzgruppe darstellt,

in der

$R^{18}$ und $R^{19}$ gleich oder verschieden sind und Wasserstoff, Niederalkyl oder Aryl bedeuten,

umsetzt,

dann (a) entweder zuerst die Thiol-Schutzgruppe in Gegenwart von Alkalimetall und Ammoniak und/oder Amin abspaltet und dann den Acylrest hydrolysiert,

oder (b) zuerst den Acylrest und dann die Thiol-Schutzgruppe in Gegenwart von Alkalimetall und Ammoniak und/oder Amin abspaltet, und dann die erhaltenen 2-Amio-3-pyrimidinthiole mit der α-Halogencarbonylverbindung in Gegenwart einer Base umsetzt.

Die erfindungsgemäße Herstellung der 2-Amio-3-pyridinthiole kann durch das folgende Formelschema erläutert werden:

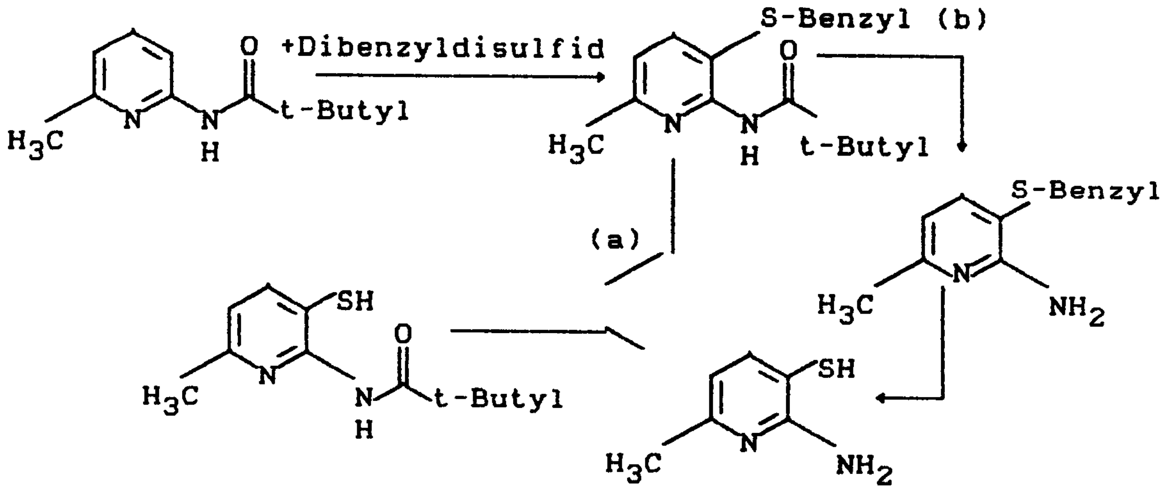


Die acylierten Pyridinamine für das erfindungsgemäße Verfahren sind an sich bekannt (J. Org. Chem. <u>48</u>, 3401, (1983)) und können beispielsweise durch Acylierung des entsprechenden Pyridinamins hergestellt werden.

Als Acylreste seien Reste der Formeln

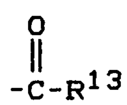 oder 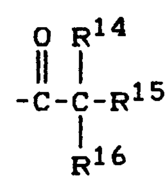 oder 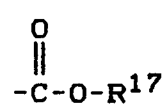

wobei

$R^{13}$ für gegebenenfalls substituiertes Phenyl und

$R^{14}$ bis $R^{15}$ gleich oder verschieden sind und für Niederalkyl oder gegebenenfalls substituiertes Phenyl stehen.

Phenyl kann beispielsweise durch Methyl, Ethyl, Propyl oder Isopropyl substituiert sein.

Niederalkyl steht im allgemeinen für einen geradkettigen oder verzweigten Kohlenwasserstoffrest mit 1 bis etwa 6 Kohlenstoffatomen.

Beispielsweise seien die folgenden acylierten Pyridinamine genannt:

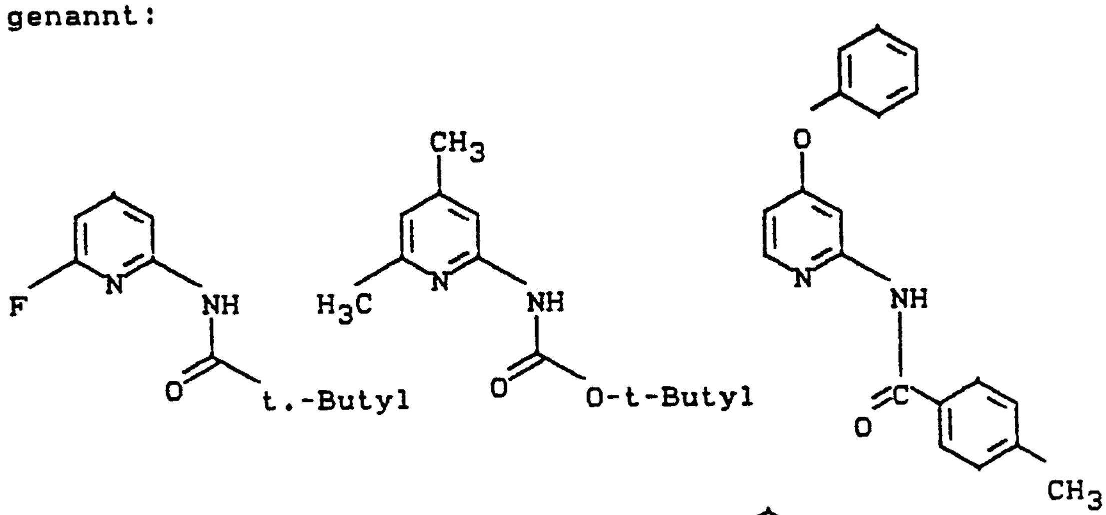

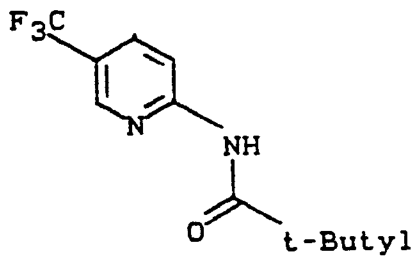

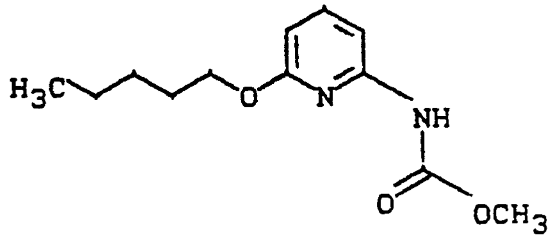

Disulfide für das erfindungsgemäße Verfahren sind an sich bekannt (Houben-Weyl, Bd. IX, 1955) und können beispielsweise durch Umsetzung von Mercaptanen mit Jod hergestellt werden.

Bei den Disulfiden für das erfindungsgemäße Verfahren steht Aryl im allgemeinen für einen Rest mit 6 bis 12 Kohlenstoffatomen, bevorzugt für einen gegebenenfalls substituierten (Methyl, Ethyl, Propyl oder iso-Propyl sowie Methoxy, Ethoxy, Propoxy oder iso-Propoxy) Phenylrest. Insbesonders bevorzugt ist Phenyl.

Niederalkyl steht im allgemeinen für einen geradkettigen oder verzweigten Kohlenwasserstoffrest mit 1 bis etwa 6 Kohlenstoffatomen.

Beispielsweise seien die folgenden Disulfide genannt:

Dibenzyldisulfid, Di-(p-methoxy-benzyl)-disulfid, Di-(p-tolyl)-disulfid, Di-benzhydryl-disulfid, Bis-(α,α-dimethyl-benzyl)disulfid.

Metallorganische Verbindungen sind im wesentlichen Alkyl-($C_1$-$C_6$) und/oder Aryl($C_6$-$C_{12}$)-Lithiumverbindungen. Bevorzugte metallorganische Verbindungen sind n-, tert.-Butyl- und Phenyl-lithium.

Als Lösungmittel für diese Verfahrensstufe seien gegenüber Organolithium inerte Solventien genannt. Bevorzugte Solventien sind Ether, wie Diethylether, 1,2-Dimethoxyethan und Tetrahydrofuran. Bevorzugtes Lösungsmittel ist Tetrahydrofuran.

Das erfindungsgemäße Verfahren kann durchgeführt werden in Gegenwart von einem oder mehreren gegenüber metallorganischen Verbindungen inerten Lösungsmittel.

Die Umsetzung der acylierten Aminopyrimidine mit den Disulfiden nach dem erfindungsgemäßen Verfahren kann im Temperaturbereich von -100° C bis +50° C, vorzugsweise im Temperaturbereich von -80° C bis +25° C, durchgeführt werden.

Es ist zweckmäßig, unter Ausschluß von Sauerstoff und Feuchtigkeit zu arbeiten. Daher wird das erfindungsgemäße Verfahren vorzugsweise unter Verwendung getrockneter Schutzgase wie Stickstoff, Helium oder Argon durchgeführt.

Das erfindungsgemäße Verfahren wird im allgemeinen unter Normaldruck durchgeführt. Es ist jedoch auch möglich, das Verfahren unter vermindertem oder erhöhtem Druck (beispielsweise im Druckbereich 0,5 bis 10,0 bar) auszuführen.

Die erste Verfahrensstufe der besonderen Ausführungsform des erfindungsgemäßen Verfahrens wird im allgemeinen wie folgt durchgeführt:

Pro Äquivalent des acylierten 2-Pyridinamins setzt man im allgemeinen 2 - 3 Äquivalente der metallorganischen Verbindung und 1 - 3 Äquivalente des Disulfids ein. Dabei werden sowohl die metallorganischen Verbindungen als auch das Disulfid vorzugsweise in gelöster Form in das Reaktionsgemisch eingetragen.

Als Solventien für das Disulfid werden vorzugsweise inerte Ether wie Diethylether, 1,2-Dimethoxyethan und, besonders bevorzugt, Tetrahydrofuran, verwendet.

Auch die Zugabe des Disulfids in fester Form und in größeren als oben genannten Überschüssen ist möglich und unter Umständen zweckmäßig.

Als Solventien für die metallorganischen Verbindungen kommen inerte Lösungsmittel, vorzugsweise Kohlenwasserstoffe, besonders bevorzugt n-Hexan und n-Pentan in Frage.

Das Produkt der ersten Reaktionsstufe kann nach üblichen Methoden isoliert werden. Es ist jedoch auch möglich, das Reaktionsprodukt direkt weiterzuverarbeiten.

Die zweite Stufe der besonderen Ausführungsform des erfindungsgemäßen Verfahrens kann in zwei Varianten durchgeführt werden.

Nach der Variante (a) spaltet man zuerst die Thiol-Schutzgruppe in Gegenwart von Alkalimetall und Ammoniak und/oder Aminen in wasserfreiem Medium ab und hydrolysiert dann den Acylrest.

Nach der Variante (b) entfernt man zuerst den Acylrest und spaltet dann die Thiol-Schutzgruppe in Gegenwart von Alkalimetallen und Ammoniak und/oder Aminen ab.

Zur Abspaltung der Thiol-Schutzgruppe nach Variante (a) verwendet man im allgemeinen Alkalimetall wie Lithium, Natrium oder Kalium, bevorzugt Natrium. Zur Reaktion gebracht werden bevorzugt pro Äquivalent des Einsatzproduktes etwa 2 - 8 Äquivalente des Metalls, insbesondere bevorzugt etwa 4 Äquivalente.

Im allgemeinen verwendet man Amine, bevorzugt primäre und/oder sekundäre Alkyl($C_1$-$C_6$)amine oder flüssiges Ammoniak für die Umsetzung. Besonders bevorzugt wird die Umsetzung in flüssigem Ammoniak durchgeführt.

Es ist jedoch auch möglich, Gemische von Lösungsmitteln einzusetzen, beispielsweise verflüssigtes Ammoniak und Amine.

Die Reaktionstemperatur liegt im allgemeinen im Bereich von -100 bis +50° C, bevorzugt im Bereich von -80 bis +20° C. Es ist zweckmäßig, unter Ausschluß von Sauerstoff und Feuchtigkeit zu arbeiten.

Das erfindungsgemäße Verfahren kann unter Normaldruck durchgeführt werden; es ist jedoch auch möglich, das Verfahren unter vermindertem oder erhöhtem Druck (beispielsweise im Druckbereich von 0,5 bis 10,0 bar) durchzuführen.

Nach erfolgter Umsetzung kann die Reaktion dadurch beendet werden, daß man feste Ammoniumsalze (z.B. Ammoniumchlorid) zusetzt und das Solvens anschließend entfernt. Die Entfernung des Solvens wird bevorzugt durch Abdampfen bei Normaldruck oder unter reduziertem Druck durchgeführt.

Zur Abspaltung des Acylrestes wird der Rückstand mit einer wässrigen Alkalimetallhydroxidlösung, vorzugsweise mit 5 - 15 %iger wäßriger Natrium- oder Kaliumhydroxidlösung, behandelt. Es kann zweckmäßig sein, dem Reaktionsgemisch zur Lösungsvermittlung geeignete organische Lösungsmittel zuzusetzen, vorzugsweise niedere Alkohole oder Amine, besonders bevorzugt Methanol oder Ethanol.

Die Temperatur bei der Hydrolyse liegt im allgemeinen im Bereich von 0 bis 200° C, bevorzugt im Bereich von 60 bis 100° C.

Es kann auch hier zweckmäßig sein, unter Ausschluß von Sauerstoff zu arbeiten.

Die Hydrolyse wird im allgemeinen unter Normaldruck durchgeführt; es ist jedoch auch möglich, die Hydrolyse unter vermindertem oder erhöhtem Druck (beispielsweise im Druckbereich von 0,5 bis 10,0) durchzuführen.

Der Zusatz reduzierender organischer oder anorganischer Substanzen während der Abspaltung des Acylrestes, bevorzugt geeigneter anorganischer reduzierender Salze, besonders bevorzugt Natriumdithionit, kann vorteilhaft sein, um eine Oxidation der Thiole zu Disulfiden zu verhindern.

Die Isolierung der substituierten 2-Amino-3-pyridinthiole erfolgt in an sich bekannter Weise.

Die Abspaltung der Acylgruppen nach Variante (b) kann durch Behandlung mit Hydrazin durchgeführt werden. Hierzu setzt man das Reaktionsprodukt der ersten Stufe mit Hydrazin oder, bevorzugt, mit Hydrazinhydrat um.

Im Rahmen des erfindungsgemäßen Verfahrens kann die Hydrazinolyse auch unter Zusatz von Wasser sowie geeigneter organischer Lösungs- und Verdünnungsmittel wie Alkoholen oder Dioxan durchgeführt werden. Dabei ist es auch möglich, Gemische verschiedener Lösungsmittel zu verwenden.

Die Umsetzungstemperatur der Hydrazinolyse liegt im allgemeinen im Bereich von 40 bis 200$^0$C, bevorzugt im Bereich von 60 bis 140$^0$C.

Die Hydrazinolyse wird im allgemeinen bei Normaldruck durchgeführt; sie kann aber auch bei vermindertem oder erhöhtem Druck (beispielsweise im Druckbereich von 0,5 bis 10,0 bar) durchgeführt werden.

Alternativ kann die Acylgruppe auch in Gegenwart von Alkali (bevorzugt Natrium- oder Kaliumhydroxid) abgespalten werden. Hierbei setzt man das Reaktionsprodukt der ersten Stufe mit einer wässrigen Alkalimetall-Hydroxidlösung, vorzugsweise mit 10 bis 30 Gew.-%iger wäßriger Natrium-oder Kaliumhydroxidlösung, um. Dabei kann es zweckmäßig sein, dem Reaktionsgemisch zur Lösungsvermittlung geeignete organische Lösungsmittel, bevorzugt niedere Alkohle($C_1$ bis $C_6$), besonders bevorzugt Methanol oder Ethanol, zuzusetzen.

Die Reaktionstemperatur bei der basischen Hydrolyse liegt im allgemeinen im Bereich von 25 bis 100° C, vorzugsweise im Bereich von 60 bis 90° C.

Die basische Hydrolyse wird im allgemeinen bei Normaldruck durchgeführt; sie kann aber auch unter vermindertem oder erhöhtem Druck (beispielsweise im Druckbereich von 0,5 bis 10,0 bar) durchgeführt werden.

Eine weitere Möglichkeit der Abspaltung der Acylgruppe bietet das Verfahren der sauren Hydrolyse. Hierbei wird das Reaktionsprodukt der ersten Reaktionsstufe mit wässrigen Mineralsäuren erhitzt. Hierzu eignen sich beispielsweise Schwefelsäure, Salpetersäure, Bromwasserstoffsäure und, bevorzugt, Salzsäure. Es kann zweckmäßig sein, dem Reaktionsgemisch geeignete organische Lösungsmittel, beispielsweise niedere Alkohle ($C_1$ bis $C_6$), bevorzugt Methanol oder Ethanol, zuzusetzen.

Bei der sauren Hydrolyse liegt die Reaktionstemperatur im Bereich von 25 bis 200° C, bevorzugt im Bereich von 60 bis 100° C.

Die saure Hydrolyse wird im allgemeinen unter Normaldruck, sie kann jedoch auch unter vermindertem oder erhöhtem Druck (beispielsweise im Druckbereich von 0,5 bis 10,0 bar) durchgeführt werden.

Zur Demaskierung der Thiolgruppe durch Abspaltung der Schutzgruppe verwendet man bevorzugt Alkalimetalle, wie Lithium, Natrium und Kalium, besonders bevorzugt verwendet man zur Demskierung der Thiolgruppe Natrium.

Pro Äquivalent des Reaktionsproduktes der Hydrolyse setzt man im allgemeinen 2 - 8 Äquivalente, bevorzugt 3 - 6 Äquivalente, des Alkalimetalles ein.

Die Abspaltung wird im allgemeinen in Gegenwart von Aminen, wie primäre und/oder sekundäre Alkyl($C_1$-$C_6$)amine oder flüssigem Ammoniak durchgeführt. Besonders bevorzugt wird die Hydrolyse in flüssigem Ammoniak durchgeführt.

Es ist jedoch auch möglich, Gemische aus flüssigem Ammoniak und anderen geeigneten Lösungsmitteln, wie die genannten Amine, zu verwenden.

Die Abspaltung der Thiol-Schutzgruppe wird im allgemeinen im Temperaturbereich von -100 bis +50°C, bevorzugt im Temperaturbereich von -80 bis +25°C, durchgeführt. Es ist zweckmäßig, unter Ausschluß von Luftsauerstoff und Feuchtigkeit zu arbeiten.

Die Abspaltung der Thiol-Schutzgruppe wird im allgemeinen bei Normaldruck durchgeführt; es ist jedoch auch möglich, diese Reaktion unter erhöhtem oder vermindertem Druck (beispielsweise im Druckbereich von 0,5 bis 10,0 bar) durchzuführen.

Nach erfolgter Umsetzung kann die Reaktion dadurch beendet werden, daß man Ammoniumsalze, beispielsweise Ammoniumchlorid, zusetzt und anschließend das Solvens entfernt,

bevorzugt durch Abdampfen bei Normaldruck oder vermindertem Druck.

Die Isolierung der 2-Amino-3-pyridinthiole erfolgt in an sich bekannter Weise.

Die erhaltenen substituierten 2-Amino-3-pyridinthiole können auch ohne vorherige Isolierung und Reinigung zur Herstellung der erfindungsgemäßen 1-H-Pyrido-[3,2-b][1,4]-thiazine verwendet werden.

Die erfindungsgemäßen 1-H-Pyrido-[3,2-b][1,4]-thiazine sind Wirkstoffe für Arzneimittel und zur therapeutischen Behandlung von Menschen und Tieren geeignet. Sie sind Lipoxygenasehemmer und besonders zur Behandlung von entzündlichen, allergischen und asthmatischen Erkrankungen geeignet. Sie können insbesondere als Antiphlogistika, Antirheumatika, Antiarteriosklerotika, Antiasthmatika, Antiallergika, Antimetastatika und Gastroprotectiva Verwendung finden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer

Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

2-Methyl-1-H-Pyrido-[3,2-b][1,4]-thiazin-3-carbonsäure-ethylester 1

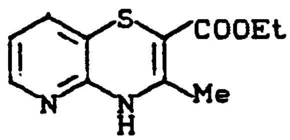

5,0 g (23.1 mmol) 2-Amino-3-(benzylthio)-pyridin werden in 150 ml verflüssigtem Ammoniak unter Rückfluß gelöst bzw. fein suspendiert, mit 2,0 g Natriummetall versetzt und etwa 45 Minuten lang bei -80° C gerührt. Man setzt bis zur Entfärbung des Reaktionsgemisches festes Ammoniumchlorid zu und läßt das Ammoniak verdunsten. Nach Trocknen des Rückstandes im Hochvakuum nimmt man in 100 ml Ethanol auf und versetzt bei ca. 10° C unter Rühren mit 3,8 g (23.1 mmol) 2-Chloracetessigsäureethylester. Nach ca. 15 Minuten stellt man das Reaktionsgemisch durch Zugabe von 50 %iger ethanolischer Essigsäure schwach sauer und erwärmt schließlich bis zur vollständigen Umsetzung auf 40 - 50° C.

Nach Abkühlen entfernt man die Hälfte des Lösungsmittels durch Abdampfen im Wasserstrahlvakuum, versetzt mit 100 $cm^3$ Eiswasser, stellt das Reaktionsgemisch durch Zugabe von Natriumhydrogencarbonat schwach alkalisch und extrahiert mit Ethylacetat oder Dichlormethan. Die

vereinigten organischen Extrakte werden über wasserfreiem Natriumsulfat getrocknet, das Lösungsmittel wird entfernt und der Rückstand umkristallisiert bzw. durch Säulenchromatographie gereinigt.

Ausbeute: 64 %
Fp.: 104 - 106° C

Analog zu Beispiel 1 erhält man:

| Bsp. | | Ausbeute (%) | Schmelzp. (Fp.; °C) |
|---|---|---|---|
| 2 | 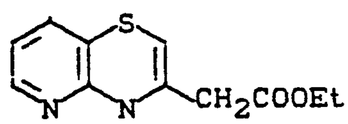 | 63 | 85 - 86 |
| 3 | 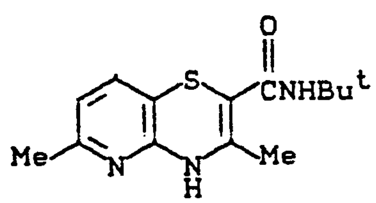 | 24 | 125 - 127 |
| 4 | 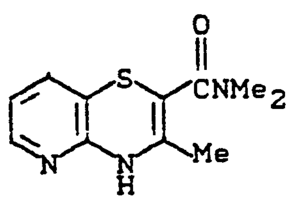 | 80 | 179 - 180 |
| 5 | 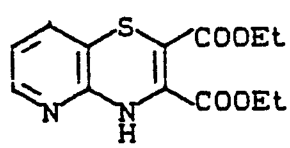 | 75 | 123 - 124 |

| | | | |
|---|---|---|---|
| 6 | 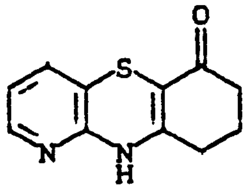 | 70 | 206 - 208 |
| 7 | 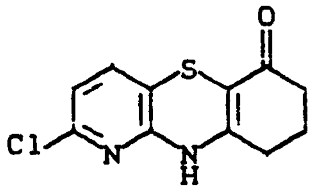 | 20 | 218 - 223 |
| 8 | 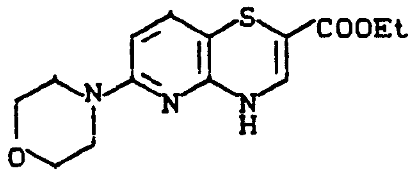 | 80 | 168 - 169 |
| 9 | 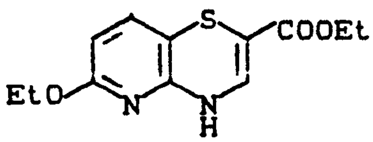 | 25 | 194 - 196 |
| 10 | 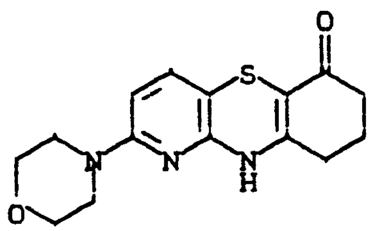 | 74 | 220 - 222 |
| 11 | 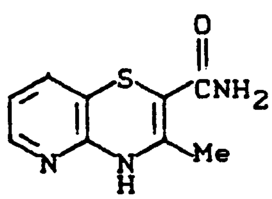 | 45 | 184 - 186 |
| 12 | 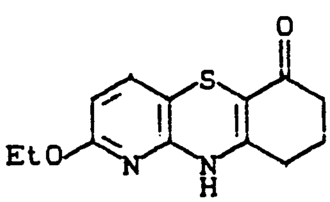 | 51 | 208 - 210 |

| | | | |
|---|---|---|---|
| 13 | 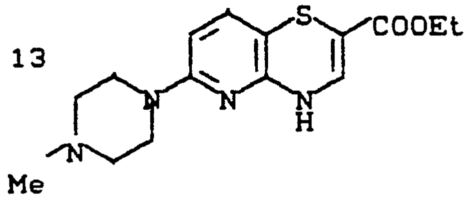 | 59 | 252 - 253 |
| 14 | 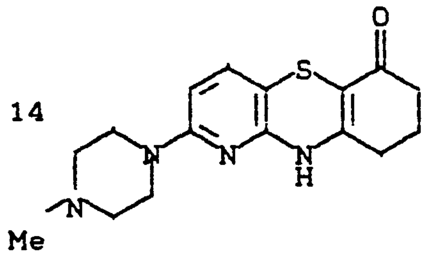 | 52 | 209 - 211 |

Beispiel 15

7-Methyl-1H-pyrido[3,2-b][1,4]-thiazin-3-carbonsäuremethylester

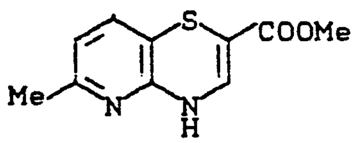

Zu 4.44 g (31.7 mmol) 2-Amino-6-methyl-3-pyridinthiol in 200 ml Methanol gibt man unter Stickstoff 1.78 g (31.7 mmol) Kaliumhydroxid und rührt etwa 30 Minuten lang bei Raumtemperatur. Nun gibt man bei 10°C unter Rühren eine Lösung von 4.8 g (35,2 mmol) 2-Chlorformylessigsäuremethylester in 70 ml Methanol zu. Man erwärmt 2 Stunden lang auf 50°C und arbeitet wie in Beispiel 1 beschrieben auf.

Ausbeute: 45 %
Fp.: 178 - 180°C

Analog zu Beispiel 15 erhält man:

| | | | | |
|---|---|---|---|---|
| 16 | 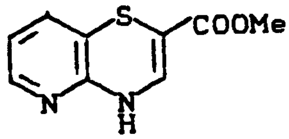 | 42 | 178 - 180 | |
| 17 | 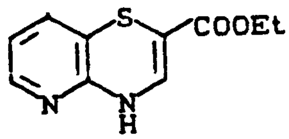 | 55 | 130 - 132 | X |
| 18 | 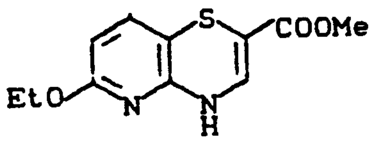 | 59 | 206 - 208 | |
| 19 | 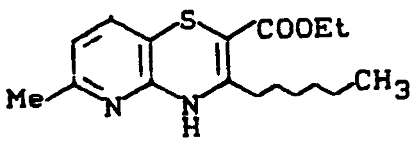 | 43 | 102 | X |
| 20 | 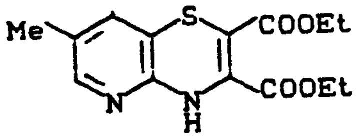 | 75 | 134 - 135 | X |
| 21 | 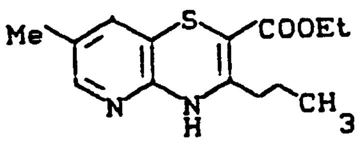 | 82 | 122 - 123 | |
| 22 | 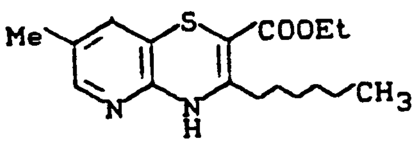 | 34 | 88 | |

Anwendungsbeispiele

Der Nachweis der lipoxygenasehemmenden Eigenschaften der 1H-Pyrido[3,2-b][1,4]thiazine der allgemeinen Formel (I) erfolgt analog den Methoden von

P. Borgeat und B. Samuelsson, Proc. Nat. Acad. Sci. 76 2148 - 2152 (1979) und

J. G. Hamilton und R. J. Karol, Progr. Lipid Res. 21 155 - 170 (1982).

Polymorphkernige Rattenleukozyten wurden aus dem Peritonealraum von Wistar-Ratten, 18 Stunden nach i. p.-Applikation von 6 ml einer 12 %igen Natriumcaseinat-Suspension, gewonnen.

Als Maß für die Lipoxygenase-Hemmung wurde die Freisetzung von Leukotrien $B_4$ an polymorphkernigen Granulozyten nach Zugabe von Substanzen und Calciumionophor mittels HPLC bestimmt.

Nach Zentrifugation und Waschen der PMNL mit Inkubationspuffer (137 mM NaCl; 2.7 mM KCl; 5.0 mM $Na_2HPO_4$; 5.55 mM Glucose; 2.0 mM $CaCl_2$ pH=7.2) wurde die Zelldichte auf 2 x $10^7$ $ml^{-1}$ eingestellt (Coulter Counter) und 1 ml dieser Zellsuspension mit 2.5 µl DMSO bzw. 2.5 µl verschiedener Testsubstanzkonzentrationen in DMSO über 5 min bei 37°C vorinkubiert. Nach Stimulation der Zellen mit 2.5 µl Calcium-Ionophor A 23187 (1 mg $ml^{-1}$ in DMSO) wurde die 6-minütige Hauptinkubation durch Zugabe von 1.5 ml $PGB_2$-haltigem Methanol gestoppt (1µg $ml^{-1}$), 2 ml-zell

freier Überstand durch Zentrifugation (1000 g, 3 min, RT) gewonnen und nach Ansäuern mit 1 n HCl auf pH 3.0 zweimal mit 4 ml Ether extrahiert. Die vereinigten Etherphasen wurden mit 4 ml Wasser (bidest.) gewaschen, unter Stickstoff-Begasung getrocknet und in 80 µl Methanol aufgenommen. Von jeder der so aufbereiteten Proben wurden 20 ul auf eine Fertigsäule (Nucleosil, Typ 7.5 C 18; 4 x 25 mm) aufgetragen und bei einer Durchflußrate von 1 ml/min (Kontrom Pumpensystem 600) chromatographiert, wobei als mobile Phase Methanol, $H_2O$ und Essigsäure (75:25:0,01) diente. Die Detektion erfolgte bei 280 nm (Uvicon 720 LC). Die Bildung des Metaboliten wurde mit Hilfe des internen Standards Protacylandin $B_2$ quantifiziert und eine Hemmung als Prozent der Kontrollen ermittelt.

Wie aus der nachfolgenden Tabelle ersichtlich ist, bewirken die erfindungsgemäßen 1H-Pyrido[3,2-b][1,4]thiazine der allgemeinen Formel (I) eine signifikante Hemmung der $LTB_4$ Biosynthese in Rattengranulozyten.

Hemmung der Leukotrien-Biosynthese

| Verbindung aus Bsp. Nr. | | g $ml^{-1}$ |
|---|---|---|
| 1 | 47 % | $2.4 \times 10^{-6}$ |
| 15 | 65 % | $2.2 \times 10^{-7}$ |
| 16 | 98 % | $2.1 \times 10^{-6}$ |
| 5 | 63 % | $2.9 \times 10^{-6}$ |

Die antiasthmatische Wirkung der erfindungsgemäßen Verbindungen kann ebenfalls nach bereits bekannten Methoden nachgewiesen werden (vgl. Samuelsson et al., FEBS Lett. 110 213 (1980) und Yen et al., Agents and Actions 10 274 (1980)).

## Patentansprüche

1. 1-H-Pyrido-[3,2-b][1,4]-thiazine der Formel

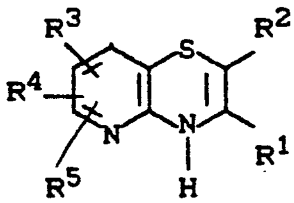

(I),

in der

$R^1$ Wasserstoff, gegebenenfalls substituiertes Alkyl oder Alkenyl oder die Gruppe

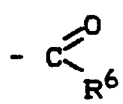

in der $R^6$ für gegebenenfalls substituiertes Alkyl, Aryl oder Alkoxy steht,

bedeutet,

$R^2$ Wasserstoff, Nitril, die Gruppe

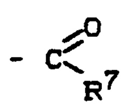

in der $R^7$ für gegebenenfalls substituiertes Alkyl oder Aryl steht,

oder die Gruppe

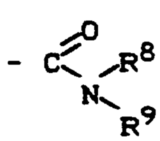

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Heterocyclus verbunden sein können,

oder die Gruppe

$$-\overset{\overset{\displaystyle O}{\|}}{C}-R^{10}$$

in der

$R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

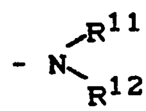

in der

$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen gegebenenfalls substituierten Heterocyclus verbunden sein können,

bedeuten

und deren Salze.

2. 1H-Pyrido[3,2-b][1,4]thiazine nach Anspruch 1,

worin

$R^1$ Wasserstoff, gegebenenfalls substituiertes Alkyl ($C_1$ bis $C_8$) oder Alkenyl ($C_2$ bis $C_8$) oder die Gruppe

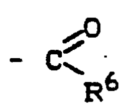

in der $R^6$ für gegebenenfalls substituiertes Alkyl ($C_1$ bis $C_8$), Alkoxy ($C_1$ bis $C_8$) oder Aryl ($C_6$ bis $C_{12}$) steht,

bedeutet,

$R^2$ Wasserstoff, Nitril, die Gruppe

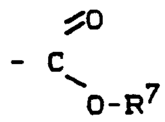

in der $R^7$ für gegebenenfalls substituiertes Alkyl ($C_1$ bis $C_8$) steht,

oder die Gruppe

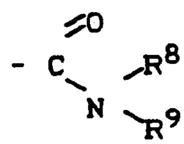

in der

$R^8$ und $R^9$ gleich oder verschieden sind und für Wasserstoff, gegebenenfalls substituiertes Alkyl ($C_1$ bis $C_8$) oder Aryl ($C_6$ bis $C_{12}$) stehen und wobei die Reste durch eine Kohlenwasserstoffbrücke, welche gegebenenfalls durch ein oder zwei Sauerstoff- und/oder Stickstoff-und/oder Schwefelatome unterbrochen sein kann, zu einem 5- oder 6-gliedrigen, gegebenenfalls substituierten Heterocyclus verbunden sein können,

oder die Gruppe

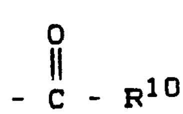

in der $R^{10}$ für gegebenenfalls substituiertes Alkyl ($C_1$ bis $C_8$) steht,

wobei $R^1$ und $R^{10}$ einen gegebenenfalls substituierten 5- bis 8-gliedrigen Carbocyclus bilden können,

oder gegebenenfalls substiutiertes Aryl ($C_6$ bis $C_{12}$) bedeutet,

$R^3$ Wasserstoff bedeutet,

und

$R^4$ und $R^5$ gleich oder verschieden sind und Wasserstoff, Halogen, gegebenenfalls substituiertes Alkyl ($C_1$ bis $C_8$), Alkoxy ($C_1$ bis $C_8$), Aryloxy ($C_6$ bis $C_{12}$), Alkylthio ($C_1$ bis $C_8$), Arylthio ($C_6$ bis $C_{12}$) oder die Gruppe

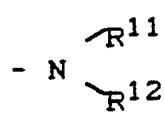

in der

$R^{11}$ und $R^{12}$ gleich oder verschieden sind und für Wasserstoff, gegebenenfalls substituiertes Alkyl ($C_1$ bis $C_8$) oder Aryl ($C_6$ bis $C_{12}$) stehen und wobei die Reste durch eine Kohlenwasserstoffbrücke, welche gegebenenfalls durch ein oder zwei Sauerstoff- und/oder Stickstoff und/oder Schwefelatome unterbrochen sein kann, zu einem 5- oder 6-gliedrigen

gegebenenfalls substituierten Heterocyclus verbunden sein können,

bedeuten

und deren Salze.

3. 1H-Pyrido-[3,2-b][1,4]-thiazine nach den Ansprüchen 1 und 2, worin

$R^1$ Wasserstoff, gegebenenfalls durch Carboxymethyl oder Carboxyethyl substituiertes Niederalkyl, Vinyl oder Carboxymethyl oder Carboxyethyl bedeutet,

$R^2$ Wasserstoff, Nitril, Carboxymethyl, Carboxyethyl oder die Gruppe

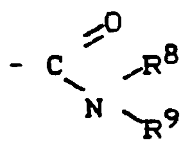

in der

$R^8$ und $R^9$ gleich oder verschieden sind und für Wasserstoff, Niederalkyl, Phenyl stehen und wobei die Reste zu einem Piperidyl-, Morpholinyl-, Piperazinyl- oder N-Methylpiperazinylrest verbunden sein können,

oder die Gruppe

$$-\overset{\overset{\displaystyle O}{\|}}{C}-R^{10}$$

in der

$R^{10}$ Methyl oder Ethyl bedeuten und

wobei $R^1$ und $R^{10}$ einen gegebenenfalls durch eine oder zwei Methylgruppen substituierten 5- bis 8-gliedrigen Carbocyclus bilden können,

bedeuten,

$R^3$ und $R^4$ Wasserstoff, Niederalkyl oder Halogen bedeuten,

und

$R^5$ Wasserstoff, Fluor, Chlor, Brom, Niederalkyl, Niederalkoxy, Piperidyl, Morpholinyl oder N-Methylpiperazinyl bedeutet,

und deren Salze.

4. Verfahren zur Herstellung von 1-H-Pyrido-[3,2-b]-[1,4]-thiazine, dadurch gekennzeichnet, daß man 2-Amino-3-pyridinthiole der Formel

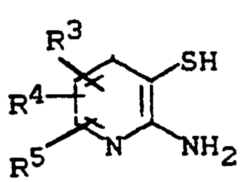

(II),

in der

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

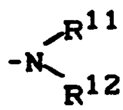

in der

$R^{11}$ und $R^{12}$ gleich oder verschieden sind und Wasserstoff, gegebenenfalls substituiertes Alkyl oder Aryl stehen, und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Heterocyclus verbunden sein können,

bedeuten,

mit einer α-Halogencarbonylverbindung der Formel

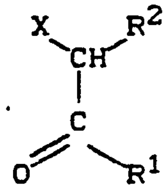

(III),

in der

X Halogen bedeutet,

$R^1$ Wasserstoff, gegebenenfalls substituiertes Alkyl oder Alkenyl oder die Gruppe

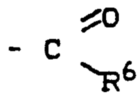

in der $R^6$ für gegebenenfalls substituiertes Alkyl, Alkoxy oder Aryl steht,

bedeutet und

$R^2$ Wasserstoff, Nitril, die Gruppe

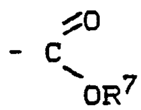

in der $R^7$ für gegebenenfalls substituiertes Alkyl oder Aryl steht,

oder die Gruppe

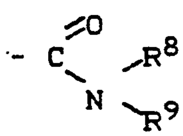

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke die gegebenenfalls noch durch weitere Heteroatome unterbrochen sein kann, zu einem 5- oder 6-gliedrigen, gegebenenfalls substituierten Heterocyclus verbunden sein können,

oder die Gruppe

$$-\overset{\overset{\displaystyle O}{\|}}{C}-R^{10}$$

in der $R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

in Gegenwart einer Base umsetzt.

5. Verfahren nach Anspruch 4, dadurch gekennzeichnet, daß es im Temperaturbereich von $-10^0$ bis $+100^0C$ durchgeführt wird.

0227982

6. Verfahren nach den Ansprüchen 4 und 5, dadurch gekennzeichnet, daß man als Base Ammonium-, Alkali- oder Erdalkali-hydroxide oder Carbonate oder organisches Amin einsetzt.

7. Verfahren nach den Ansprüchen 4 bis 6, dadurch gekennzeichnet, daß man zuerst 2-Pyridinamine der Formel

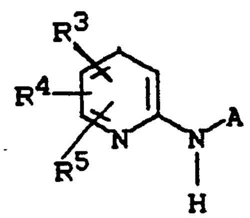

(IV),

in der

A für einen Acylrest der Formel

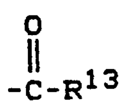 oder 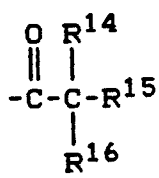 oder 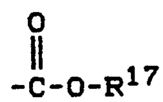

wobei

$R^{13}$ für gegebenenfalls substituiertes Phenyl steht und

$R^{14}$ bis $R^{17}$ gleich oder verschieden sind und für Niederalkyl oder gegebenenfalls substituiertes Phenyl stehen,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

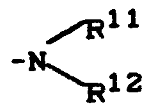

in der

$R^{11}$ und $R^{12}$ gleich oder verschieden sind und Wasserstoff, gegebenenfalls substituiertes Alkyl oder Aryl stehen, und wobei die Reste durch eine Kohlenwasserstoffbrücke, die gegebenenfalls durch weitere Heteroatome unterbrochen sein kann, zu einem 5-oder 6-gliedrigen, gegebenenfalls substituierten Heterocyclus verbunden sein können,

bedeuten,

in Gegenwart von metallorganischen Verbindungen und Disulfiden der Formel

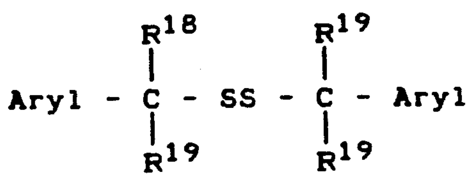

(V),

in der

der Rest 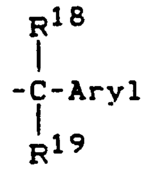 eine Thiol-Schutzgruppe darstellt,

in der

$R^{18}$ und $R^{19}$ gleich oder verschieden sind und Wasserstoff, Niederalkyl oder Aryl bedeuten,

umsetzt,

dann (a) entweder zuerst die Thiol-Schutzgruppe in Gegenwart von Alkalimetall und Ammoniak und/oder Amin abspaltet und dann den Acylrest hydrolysiert,

oder (b) zuerst den Acylrest und dann die Thiol-Schutzgruppe in Gegenwart von Alkalimetall und Ammoniak und/oder Amin abspaltet, und dann die erhaltenen 2-Amio-3-pyrimidithiole mit der α-Halogencarbonylverbindung in Gegenwart einer Base umsetzt.

8. 1-H-Pyrido-[3,2-b][1,4]-thiazine der Formel

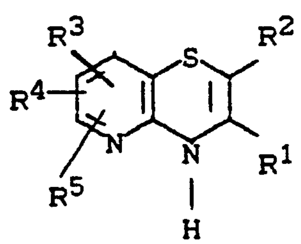

(I),

in der

$R^1$ Wasserstoff, gegebenenfalls substituiertes Alkyl oder Alkenyl oder die Gruppe

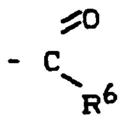

in der $R^6$ für gegebenenfalls substituiertes Alkyl, Aryl oder Alkoxy steht,

bedeutet,

$R^2$ Wasserstoff, Nitril, die Gruppe

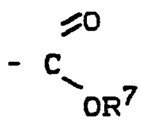

in der $R^7$ für gegebenenfalls substituiertes Alkyl oder Aryl steht,

oder die Gruppe

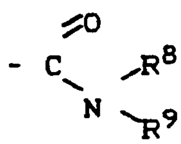

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Hetereocyclus verbunden sein können,

oder die Gruppe

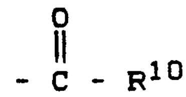

in der $R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

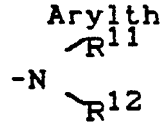

in der

$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann,

zu einem 5- oder 6-gliedrigen, gegebenenfalls substituierten Heterocyclus verbunden sein können,

bedeuten

und deren Salze, zur therapeutischen Anwendung.

9. Arzneimittel, enthaltend als Wirkstoff ein oder mehrere 1H-Pyrido[3,2-b][1,4]thiazine der Formel I

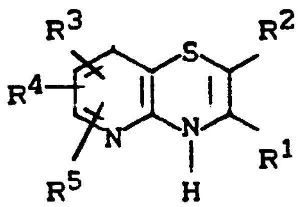

(I),

in der

$R^1$ Wasserstoff, gegebenenfalls substituiertes Alkyl oder Alkenyl oder die Gruppe

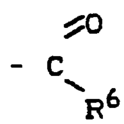

in der $R^6$ für gegebenenfalls substituiertes Alkyl, Aryl oder Alkoxy steht,

bedeutet,

$R^2$ Wasserstoff, Nitril, die Gruppe

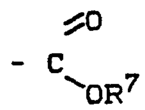

in der $R^7$ für gegebenenfalls substituiertes Alkyl oder Aryl steht,

oder die Gruppe

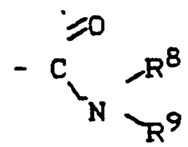

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke , die gegebenenfalls noch durch weitere Heteroatome unterbrochen sein kann, zu einem 5- bis 6-gliedrigen, gegebenenfalls substituierten Heterocyclus verbunden sein können,

oder die Gruppe

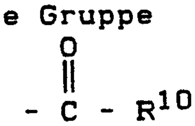

in der $R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substiutiertes Aryl bedeutet,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

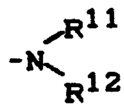

in der

$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen gegebenenfalls substituierten Heterocyclus verbunden sein können,

bedeuten,

und/oder deren Salze.

10. Verwendung von 1-H-Pyrido-[3,2-b][1,4]-thiazinen der Formel

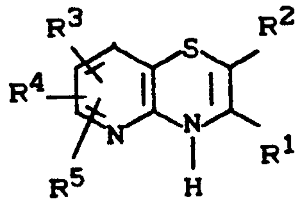

(I),

in der

$R^1$ Wasserstoff, gegebenenfalls substituiertes Alkyl oder Alkenyl oder die Gruppe

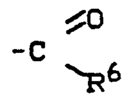

in der $R^6$ für gegebenenfalls substituiertes Alkyl, Aryl oder Alkoxy steht,

bedeutet,

$R^2$ Wasserstoff, Nitril, die Gruppe

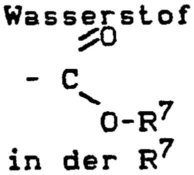

in der $R^7$ für gegebenenfalls substituiertes Alkyl oder Aryl steht,

oder die Gruppe

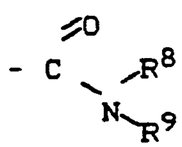

in der

$R^8$ und $R^9$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen, gegebenenfalls substituierten Heterocyclus verbunden sein können,

oder die Gruppe

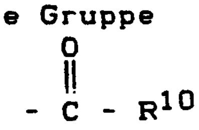

in der

$R^{10}$ für gegebenenfalls substituiertes Alkyl steht,

wobei $R^1$ und $R^{10}$ einen gegebenenfalls substituierten 5- bis 8-gliedrigen Carbocyclus bilden können,

oder gegebenenfalls substituiertes Aryl bedeutet,

und

$R^3$, $R^4$ und $R^5$ gleich oder verschieden sind und Wasserstoff, Halogen, gegebenenfalls substituiertes Alkyl, Alkoxy, Aryloxy, Alkylthio, Arylthio oder die Gruppe

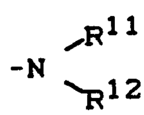

in der

$R^{11}$ und $R^{12}$ gleich oder verschieden sind und für Wasserstoff, gegebenenfalls substituiertes Alkyl oder Aryl stehen und wobei die Reste durch eine Kohlenwasserstoffbrücke, die gegebenenfalls noch durch weitere Heteroatome unterbrochen sein kann, zu einem 5- oder 6-gliedrigen gegebenenfalls substituierten Heterocyclus verbunden sein können,

bedeuten

und/oder deren Salze zur Herstellung von Arzneimitteln.

Europäisches Patentamt

# EUROPÄISCHER RECHERCHENBERICHT

0227982
Nummer der Anmeldung

EP 86 11 7011

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 100 527 (BAYER)<br>* Patentansprüche 1,5 *<br>--- | 1,9 | C 07 D 513/04<br>A 61 K 31/54 //<br>(C 07 D 513/04<br>C 07 D 279:00<br>C 07 D 221:00 ) |
| A | EP-A-0 101 898 (BAYER)<br>* Patentansprüche 1,6 *<br>----- | 1,9 | |
| | | | RECHERCHIERTE SACHGEBIETE (Int. Cl.4) |
| | | | C 07 D 513/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 23-03-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82